(19) Europäisches Patentamt
European Patent Office
Office européen des brevets

(11) Veröffentlichungsnummer : **0 104 146**
**B1**

(12) # EUROPÄISCHE PATENTSCHRIFT

(45) Veröffentlichungstag der Patentschrift : **30.07.86**

(51) Int. Cl.⁴ : **G 03 C 7/26**// C07D211/46, C07D211/58

(21) Anmeldenummer : **83810415.6**

(22) Anmeldetag : **15.09.83**

(54) **Farbphotographisches Aufzeichnungsmaterial.**

(30) Priorität : **21.09.82 CH 5578/82**

(43) Veröffentlichungstag der Anmeldung : **28.03.84 Patentblatt 84/13**

(45) Bekanntmachung des Hinweises auf die Patenterteilung : **30.07.86 Patentblatt 86/31**

(84) Benannte Vertragsstaaten : **BE CH DE FR GB IT LI NL**

(56) Entgegenhaltungen :
**EP-A- 0 011 051**
**DE-A- 2 126 954**
**FR-A- 2 334 672**
**FR-A- 2 351 961**
**FR-A- 2 366 278**
**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Klybeckstrasse 141**
**CH-4002 Basel (CH)**

(72) Erfinder : **Leppard, David George, Dr.**
**Route de Bourguillon 6**
**CH-1723 Marly (CH)**
Erfinder : **Rody, Jean, Dr.**
**Rütiring 82**
**CH-4125 Riehen (CH)**

**EP 0 104 146 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein farbphotographisches Aufzeichnungsmaterial, das in mindestens einer lichtempfindlichen Silberhalogenidemulsionsschicht und/oder in mindestens einer der üblichen Hilfsschichten als Stabilisator eine spezifische Polyalkylpiperidin-Verbindung enthält.

Polyalkylpiperidine sind als sterisch gehinderte Amine allgemein als Lichtschutzmittel für organische Materialien, insbesondere für Polymere, bekannt. Es wurde auch bereits in der DE-A 2 126 954 vorgeschlagen, solche Polyalkylpiperidine als Mittel gegen das Ausbleichen von Farbphotographien zu verwenden. Es wurde weiterhin in der EP-A 11 051 vorgeschlagen, als Lichtschutzmittel für Farbphotographien bestimmte Polyalkylpiperidinderivate zu verwenden, die mindestens eine Phenolgruppe enthalten. Es handelt sich dabei um Polyalkylpiperidinester von Hydroxylbenzylmalonsäuren.

In Weiterverfolgung dieser Forschungsarbeiten wurde gefunden, dass Amide aus Phenolalkancarbonsäuren und 4-Aminopolyalkylpiperidinen ebenfalls eine ausgezeichnete Lichtschutzwirkung für Farbphotographien besitzen und darüber hinaus auch im Dunkeln eine Stabilisierung der Farbstoffe gegenüber Veränderungen beim Lagern des Aufzeichnungsmaterials bewirken.

Gegenstand der vorliegenden Erfindung ist daher ein farbphotographisches Aufzeichnungsmaterial, das in mindestens einer lichtempfindlichen Siberhalogenidemulsionsschicht, einer Zwischenschicht, einer Bildempfangsschicht und/oder einer Schutzschicht als Stabilisator mindestens eine Polyalkylpiperidinverbindung der Formel I enthält,

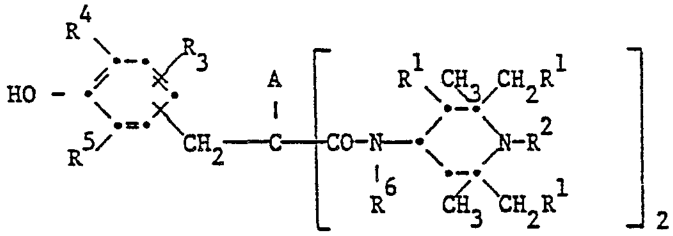

(I)

worin

$R^1$ Wasserstoff oder Methyl ist,

$R^2$ Hydroxyl, $C_1$-$C_{12}$-Alkyl, $C_3$-$C_6$-Alkenyl, $C_3$-$C_4$-Alkinyl, $C_7$-$C_{12}$-Aralkyl, Glycidyl, durch Halogen, Cyan, —$COOR^7$ oder —$CON(R^8)(R^9)$ substituiertes $C_1$-$C_4$-Alkyl, eine Gruppe —CO—$R^{10}$, —CO—$OR^7$, —CO—$N(R^8)(R^9)$, —$CH_2$—$CH(R^{11})$—$OR^{12}$, —SO—$R^{13}$, —SO—$R^{13}$, —$OR^7$ oder —OOC—$R^{10}$ bedeutet,

$R^3$ Wasserstoff oder Methyl ist,

$R^4$ $C_1$-$C_8$-Alkyl, $C_5$-$C_8$-Cycloalkyl, $C_7$-$C_9$-Phenylalkyl, Phenyl oder $C_7$-$C_{10}$-Alkylphenyl und

$R^5$ Wasserstoff, $C_1$-$C_8$-Alkyl, $C_5$-$C_8$-Cycloalkyl, $C_7$-$C_9$-Phenylalkyl, Phenyl oder $C_7$-$C_{10}$-Alkylphenyl bedeutet,

$R_6$ Wasserstoff, $C_1$-$C_{18}$-Alkyl, $C_3$-$C_6$-Alkenyl, $C_3$-$C_{12}$-Cycloalkyl, $C_7$-$C_{12}$-Aralkyl, $C_7$-$C_{10}$-Alkylphenyl, durch —$OR^8$, —$N(R^9)_2$ oder eine Gruppe

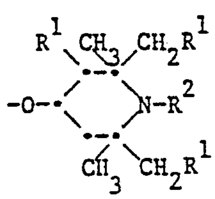

substituiertes $C_2$-$C_4$-Alkyl bedeutet,

$R^7$ $C_1$-$C_{12}$-Alkyl, Allyl, Cyclohexyl oder Benzyl bedeutet,

$R^8$ $C_1$-$C_{12}$-Alkyl, Allyl, Cyclohexyl, Benzyl oder Phenyl bedeutet,

$R^9$ Wasserstoff, $C_1$-$C_{12}$-Alkyl, Allyl, Cyclohexyl oder Benzyl bedeutet oder

$R^8$ und $R^9$ zusammen mit dem N-Atom, an das sie gebunden sind, einen 5- oder 6-gliedrigen heterocyclischen Ring bilden,

$R^{10}$ Wasserstoff, $C_1$-$C_{12}$-Alkyl, $C_2$-$C_6$-Alkenyl, Chlormethyl, $C_5$-$C_8$-Cycloalkyl, $C_7$-$C_{12}$-Aralkyl, Phenyl, $C_7$-$C_{10}$-Alkylphenyl oder durch 1 oder 2 $C_1$-$C_4$-Alkylgruppen und eine Hydroxygruppe substituiertes Phenyl Phenylmethyl oder Phenylethyl bedeutet,

$R^{11}$ Wasserstoff, $C_1$-$C_4$-Alkyl, $C_2$-$C_{13}$-Alkoxymethyl, Phenyl oder Phenoxymethyl bedeutet,

$R^{12}$ Wasserstoff, $C_1$-$C_{12}$-Alkyl, —CO—$R^{10}$ oder —CO—$N(R^8)(R^9)$ bedeutet,

$R^{13}$ $C_1$-$C_{12}$-Alkyl, Phenyl oder $C_7$-$C_{18}$-Alkylaryl bedeutet,

A Wasserstoff, $C_1$-$C_{18}$-Alkyl, durch mindestens eine der Gruppen —$OR^{14}$, —$SR^{15}$, —CN, —CO—X—$R^{16}$, —O—CO—$R^{17}$ oder —$P(O)(OR^{18})_2$ substituiertes $C_1$-$C_{10}$-Alkyl, durch —O—, —S—, —SO— oder —$SO_2$— unterbrochenes $C_2$-$C_{20}$-Alkyl, $C_3$-$C_{18}$-Alkenyl, $C_3$-$C_8$-Alkinyl, $C_3$-$C_{12}$-Cycloalkyl, $C_6$-$C_{10}$-Alkylcycloalkyl, $C_6$-$C_{10}$-Cycloalkylalkyl, $C_7$-$C_{12}$-Aralkyl, $C_8$-$C_{16}$-Alkylaralkyl, Phenyl, eine Gruppe

—CN, —CO—$R^{20}$, —$SO_2$—$R^{21}$, —P(O)($OR^{18}$) oder eine der folgenden Gruppen bedeutet

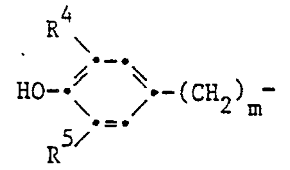

,

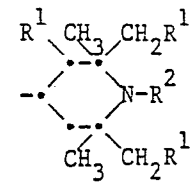

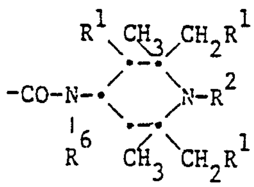

,

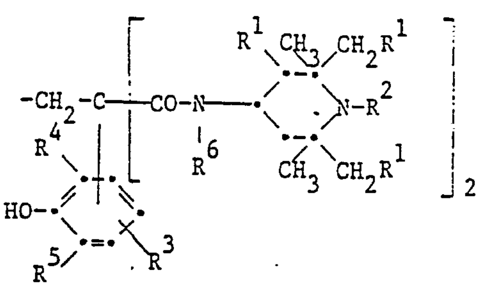

worin

X —O— oder —N($R^{19}$)— bedeutet,

$R^{14}$ Phenyl, Benzyl oder Cyclohexyl,

$R^{15}$ Phenyl oder $C_7$-$C_9$-Phenylalkyl,

$R^{16}$ $C_1$-$C^{18}$-Alkyl, $C_5$-$C_8$-Cycloalkyl und

$R^{17}$ $C_1$-$C_{18}$-Alkyl, $C_5$-$C_8$-Cycloalkyl, Phenyl, $C_7$-$C_9$-Phenylalkyl oder eine Gruppe

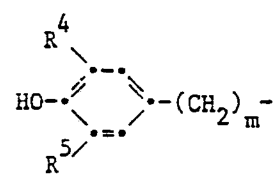

bedeutet,

$R^{18}$ $C_1$-$C_8$ Alkyl, Allyl oder Phenyl ist

$R^{19}$ Wasserstoff, $C_1$-$C_{18}$-Alkyl, Allyl, Cyclohexyl, Benzyl oder Phenyl bedeutet oder zusammen mit $R^{16}$ und dem N-Atom einen 5- oder 6-gliedrigen heterocyclischen Ring bildet,

$R^{20}$ Wasserstoff, $C_1$-$C_{18}$-Alkyl, $C_2$-$C_6$-Alkenyl, $C_5$-$C_8$-Cycloalkyl, Phenyl, $C_7$-$C_{10}$-Alkylphenyl, $C_7$-$C_9$-Phenylalkyl und

$R^{21}$ $C_1$-$C_{18}$-Alkyl, Cyclohexyl, Phenyl, Naphthyl, $C_7$-$C_{18}$-Alkylaryl, $C_7$-$C_9$-Phenylalkyl oder eine Gruppe der Formel

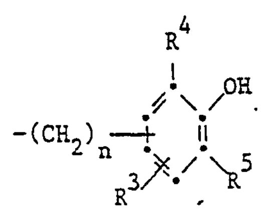

bedeutet,

m null, 1 oder 2 ist und

n null, 1, 2 oder 3 ist.

Darin können die Substituenten $R^4$, $R^5$ und $R^{18}$ $C_1$-$C_8$-Alkyl sein und sie können als solche unverzweigtes oder verzweigtes Alkyl sein, wie z. B. Methyl, Ethyl, Propyl, Isopropyl, Butyl, tert.-Butyl, Isopentyl, n-Hexyl, 2-Ethylbutyl, n-Octyl oder 1,1,3,3-Tetramethylbutyl. $R^2$, $R^7$, $R^8$, $R^9$, $R^{10}$, $R^{12}$ und $R^{13}$ als $C_1$-$C_{12}$-Alkyl können darüber hinaus auch z. B. Nonyl, Decyl, oder Dodecyl sein. $R^6$, $R^{16}$, $R^{17}$,$R^{19}$, $R^{20}$, $R^{21}$ und A als $C_1$-$C_{18}$-Alkyl können darüber hinaus auch z. B. Tetradecyl, Hexadecyl oder Octadecyl sein.

$R^2$ und $R^6$ als $C_3$-$C_6$-Alkenyl können z. B. Allyl, Methallyl, 3,3-Dimethylallyl oder 2-Butenyl sein. $R^{10}$ und $R^{20}$ als $C_2$-$C_6$-Alkenyl können darüber hinaus auch Vinyl oder Isopropenyl sein. A als $C_3$-$C_{18}$-Alkenyl kann die Bedeutungen von $R^2$ haben und darüber hinaus noch z. B. 2-Octenyl, 11-Undecenyl oder Oleyl sein.

$R^2$ als $C_3$-$C_4$-Alkinyl kann z. B. 2-Propinyl oder 2-Butinyl sein. A als $C_3$-$C_8$-Alkinyl kann darüber hinaus auch z. B. 2-Hexinyl oder 2-Octinyl sein.

$R^4$, $R^5$, $R^{10}$, $R^{16}$, $R^{17}$ und $R^{20}$ als $C_5$-$C_8$-Cycloalkyl können z. B. Cyclopentyl, Cyclohexyl oder Cyclooctyl sein. $R^6$ und A als $C_3$-$C_{12}$-Cycloalkyl können darüber hinaus auch Cyclopropyl, Cyclobutyl, Cyclodecyl oder Cyclododecyl sein.

A als $C_6$-$C_{10}$-Alkylcycloalkyl kann z. B. Methylcyclopentyl, Mono- und Dimethylcyclohexyl oder Mono- und Dimethylcyclooctyl sein. A als $C_6$-$C_{10}$-Cycloalkylalkyl kann z. B. Cyclopentylmethyl, Cyclohexylmethyl, Cyclohexylethyl oder Cyclooctylmethyl sein.

$R^4$, $R^5$, $R^{15}$, $R^{17}$, $R^{20}$ und $R^{21}$ als $C_7$-$C_9$-Phenylalkyl z. B. Benzyl, 2-Phenylethyl, 3-Phenylpropyl oder $\alpha,\alpha$-Dimethylbenzyl sein. $R^2$, $R^6$, $R^{10}$ und A als $C_7$-$C_{12}$-Aralkyl können darüber hinaus auch z. B. Phenylbutyl oder Naphthylmethyl sein. A als Alkyl-aralkyl kann z. B. 4-Methylbenzyl, 3-tert.-Butylbenzyl, 4-Methylnaphthyl-1-methyl- oder 2-(4-Isopropylphenyl)-propyl sein.

$R^4$, $R^5$, $R^6$, $R^{10}$ und $R^{20}$ als $C_7$-$C_{10}$-Alkylphenyl können z. B. 4-Methyl-, 4-tert.-Butyl, 3-Isopropyl- oder 3,5-Dimethylphenyl sein. $R^{13}$ und $R^{21}$ als $C_7$-$C_{18}$-Alkylaryl können darüber hinaus auch z. B. 4-Octylphenyl, 4-Dodecylphenyl oder 4-Methylnaphthyl-1 sein.

$R^8$ und $R^9$ sowie $R^{19}$ und $R^{16}$ können jeweils zusammen mit dem N-Atom, an das sie gebunden sind, einen heterocyclischen Ring bilden, wie z. B. einen Pyrrolidin-, Piperidin- oder Morpholinring.

Der Substituent A kann substituiertes oder unterbrochenes Alkyl sein, wie z. B. 2-Cyanethyl, 2-Ethoxycarbonylethyl, N-Butyl-2-carbamoylethyl, 2-Methoxyethyl, 2-Phenoxypropyl, 3-Phenylthiopropyl, 2-Dodecylthioethyl, 2-Butylsulfonyl-ethyl, 2-Isopropoxypropyl, 2-Acetoxybutyl, 2-Benzoyloxyethyl oder 2-(Diethylphosphono)-ethyl.

$R^{11}$ als $C_2$-$C_{13}$-Alkoxymethyl kann z. B. Methoxy-, Ethoxy-, Isopropoxy-, Butoxy-, 2-Ethylbutoxy-, Octyloxy- oder Dodecyloxymethyl sein.

Im phenolischen Rest der Formel I kann die Hydroxylgruppe in meta- oder para-Stellung zur $CH_2$-Gruppe sein, bevorzugt ist sie in para-Stellung. Wenn die $CH_2$-Gruppe in p-Stellung zur Hydroxylgruppe ist, so ist $R^3$ in m-Stellung. Wenn die $CH_2$-Gruppe in m-Stellung ist, so ist $R^3$ in p-Stellung zur Hydroxylgruppe.

Bevorzugt verwendet man als Stabilisatoren Verbindungen der Formel II

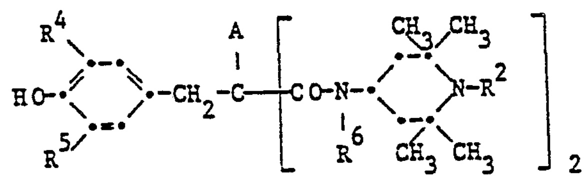

(II)

worin

$R^2$ Hydroxy, $C_1$-$C_4$-Alkyl, $C_3$-$C_4$-Alkenyl, Propargyl, Benzyl, Glycidyl, durch Halogen, Cyan, $-COOR^7$, $-CON(R^8)(R^9)$ substituiertes $C_1$-$C_4$-Alkyl, eine Gruppe $-CO-R^{10}$, $-CO-OR^7$, $-CO-N(R^8)(R^9)$, $-CH_2-CH(R^{11})-OR^{12}$, $-SO-R^{13}$, $-SO_2-R^{13}$, $-OR^7$ oder $-OOC-R^{10}$ bedeutet

$R^4$ $C_1$-$C_4$-Alkyl und $R^5$ Wasserstoff oder $C_1$-$C_4$-Alkyl ist,

$R^6$ Wasserstoff, $C_1$-$C_{12}$-Alkyl, $C_3$-$C_7$-Alkoxyalkyl, Cyclohexyl oder eine Gruppe der Formel

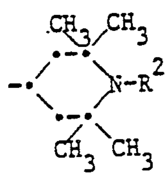

bedeutet,

$R^7$ $C_1$-$C_8$-Alkyl, Benzyl oder Cyclohexyl bedeutet,

$R^8$ $C_1$-$C_8$-Alkyl, Cyclohexyl oder Phenyl bedeutet,

$R^9$ Wasserstoff oder $C_1$-$C_8$-Alkyl bedeutet oder $R^8$ und $R^9$ zusammen mit dem N-Atom einen Piperidin- oder Morpholinring bilden,

$R^{10}$ $C_1$-$C_4$-Alkyl, $C_2$-$C_3$-Alkenyl, Chlormethyl, Phenyl, Benzyl oder eine Gruppe der Formel

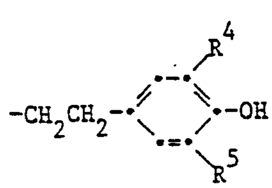

bedeutet,

$R^{11}$ Wasserstoff, Methyl oder Phenyl ist,

$R^{12}$ Wasserstoff, $C_1$-$C_8$-Alkyl, $-CO-R^{10}$ oder $-CO-N(R^8)(R^9)$ bedeutet,

$R^{13}$ $C_1$-$C_4$-Alkyl, Phenyl oder p-Tolyl bedeutet und

A Wasserstoff, $C_1$-$C_8$-Alkyl, Cyan, Allyl oder Benzyl bedeutet.

Besonders bevorzugt als Stabilisatoren sind Verbindungen der Formel II, worin

$R^2$ Hydroxy, Methyl, Allyl, Benzyl, 2-Hydroxyethyl, Acetyl, Acryloyl, Methoxy, Acetoxy oder eine Gruppe —CO—N($R^8$)($R^9$) bedeutet,

$R^4$ tert.-Butyl und $R^5$ Methyl oder tert.-Butyl ist,

$R^6$ Wasserstoff oder $C_1$-$C_8$-Alkyl ist,

$R^8$ $C_1$-$C_8$-Alkyl, Phenyl oder Cyclohexyl und $R^9$ Wasserstoff oder $C_1$-$C_8$-Alkyl ist und

A Wasserstoff, $C_1$-$C_8$-Alkyl oder Benzyl bedeutet.

Ein Teil der hier beschriebenen Verbindungen der formel I ist bekannt aus der DE-OS 26 47 452, wo die Verbindungen als Lichtschutzmittel für Kunststoffe beschrieben sind. Dort sind auch verschiedene Verfahren zur Herstellung solcher Verbindungen beschrieben. Soweit die Verbindungen neu sind, können sie in Analogie zu den beschriebenen Verbindungen hergestellt werden.

Die Synthese der Verbindungen geht vorzugsweise von N,N'-Bis(polyalkylpiperidyl-4)-malonsäurediamiden der Formel III aus, die durch Umsetzung mit einem Mol Alkali und einem Mol einer Halogenverbindung A-Hal in die Verbindungen der Formel IV übergeführt werden :

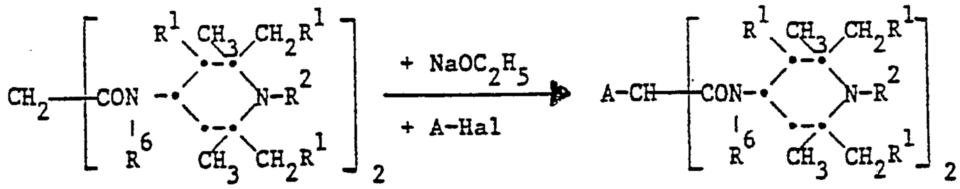


(III) (IV)

Der phenolische Rest kann in die Amide IV durch Umsetzung mit den entsprechenden Hydroxybenzyl-thiocarbamaten, -aminen oder -alkoxiden V eingeführt werden :

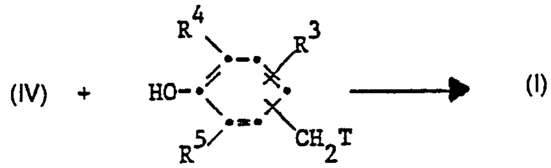


(V)

T = —S—CS—$NR_2$ oder —$NR_2$ oder —OR

R = $C_1$-$C_4$-Alkyl.

Alternativ kann man auch eine Verbindung der Formel III mit einem Mol V reagieren lassen und als zweiten Schritt den Rest A einführen.

Man kann die Reihenfolge der Reaktionsschritte beliebig abändern und z. B. ein Dialkylmalonat mit A—Hal umsetzen zum Zwischenprodukt A—CH$(COOR)_2$, das dann entweder zuerst mit einer Verbindung der Formel V und anschliessend mit einer 4-Aminopiperidinverbindung der Formel VI

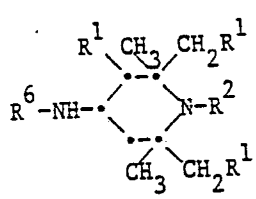

(VI)

umgesetzt wird — oder umgekehrt.

Die 4-Aminopiperidine der Formel VI sind bekannt aus den DE-A-20 40 975 und 23 49 962 und werden durch reduktive Aminierung der 4-Oxopiperidine hergestellt.

Die Einführung des Restes $R^2$ kann bereits bei der Synthese von VI erfolgen oder nach jedem der folgendem Syntheseschritte.

Sie kann nach den üblichen Methoden zur N-Substitution sekundärer Amine erfolgen, z. B. durch Reaktion mit den entsprechenden Halogenverbindungen Hal-$R^2$.

Falls $R^2$ ein Rest —$CH_2$—CH($R^{11}$)—$OR^{12}$ ist, so kann dieser durch Reaktion mit einem Oxiran

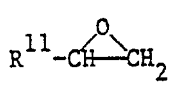

und gegebenenfalls anschliessende Alkylierung oder Acylierung der Hydroxygruppe eingeführt werden. $R^2$ als Hydroxylgruppe kann durch Reduktion der entsprechenden N-Oxyle eingeführt werden. Durch Veretherung oder Veresterung der N-Hydroxylverbindungen erhält man die Verbindungen, worin $R^2$ eine Gruppe $—OR^7$ oder $—OOC—R^{10}$ ist.

Beispiele einzelner Verbindungen der Formel I, wie sie gemäss der Erfindung als Lichtschutzmittel für farbphotographisches Aufzeichnungsmaterial verwendet werden können, sind die folgenden Verbindungen :

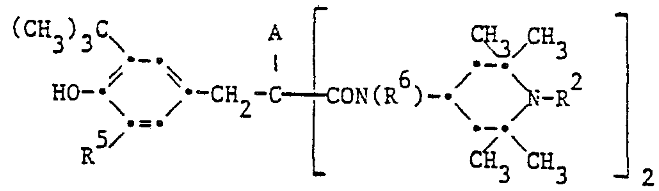

| Verbindung Nr. | $R^5$ | A | $R^6$ | $R^2$ |
|---|---|---|---|---|
| 1 | $-CH_3$ | H | $n-C_8H_{17}$ | $-CO-CH=CH_2$ |
| 2 | $-CH_3$ | H | $n-C_4H_9$ | $-CH_3$ |
| 3 | $-CH_3$ | H | H | $-CH_3$ |
| 4 | $-CH_3$ | H | $-C_2H_5$ | $-CO-CH_3$ |
| 5 | tert.-Butyl | H | H | $-CO-CH=CH_2$ |
| 6 | tert.-Butyl | $n-C_4H_9$ | H | $-CO-CH=CH_2$ |
| 7 | tert.-Butyl | H | $n-C_4H_9$ | $-CO-CH_3$ |
| 8 | tert.-Butyl | $-C_2H_5$ | H | $-CO-CH=CH_2$ |
| 9 | tert.-Butyl | $n-C_4H_9$ | H | $-CH_3$ |
| 10 | tert.-Butyl | $-C_2H_5$ | H | $-CH_2CH=CH_2$ |
| 11 | tert.-Butyl | H | $n-C_4H_9$ | $-CH_3$. |
| 12 | tert.-Butyl | H | H | $-CO-CH_3$ |
| 13 | tert.-Butyl | H | $n-C_4H_9$ | Benzyl |
| 14 | tert.-Butyl | $-CH_2CH=CH_2$ | H | $-CH_2CH=CH_2$ |
| 15 | tert.-Butyl | H | $n-C_4H_9$ | $-CO-CH=CH_2$ |
| 16 | tert.-Butyl | Benzyl | H | Benzyl |
| 17 | tert.-Butyl | Benzyl | $-CH_2CH_2OH$ | $-CH_3$ |
| 18 | tert.-Butyl | $n-C_4H_9$ | $n-C_4H_9$ | $-CO-CH=CH_2$ |
| 19 | tert.-Butyl | $n-C_4H_9$ | $n-C_4H_9$ | Benzyl |
| 20 | tert.-Butyl | $-C_2H_5$ | $n-C_6H_{13}$ | $-CH_2CH=CH_2$ |
| 21 | tert.-Butyl | $n-C_8H_{17}$ | $-CH_3$ | $-CO-N(CH_3)_2$ |
| 22 | tert.-Butyl | H | Cyclohexyl | $-CH_2CH_2OH$ |
| 23 | tert.-Butyl | H | 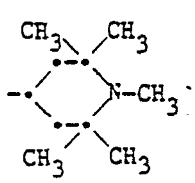 | $-CO-CH_3$ |

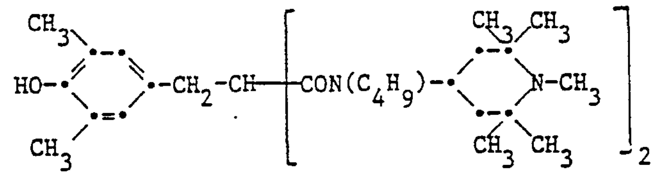

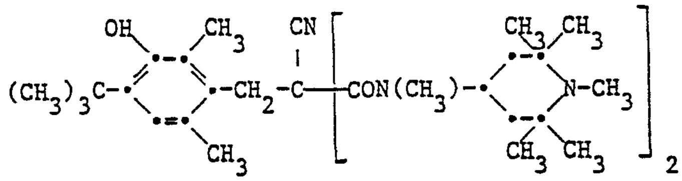

Die Verbindungen der Formel I sind in Wasser kaum löslich und sie werden deshalb den photographischen Schichten vorzugsweise als Dispersion oder Emulsion zugesetzt. In der Regel werden die Stabilisatoren zusammen mit den Farbkupplern in das photographische Material eingearbeitet. Hierzu löst man die Verbindungen der Formel I zusammen mit den Farbkupplern und gegebenenfalls mit weiteren Lichtschutzmitteln in einem niedrigsiedenden organischen Lösungsmittel, wie Methylacetat, Ethylacetat, Tetrachlorkohlenstoff, Chloroform, Methanol, Ethanol, n-Butanol, Dioxan, Aceton oder Benzol, einem hochsiedenden organischen Lösungsmittel, wie Tricresylphosphat, N,N-Diethyllauramid, Di-n-butylphthalat oder Ethyl-N-diphenylcarbamat, oder einem Lösungsmittelgemisch aus den oben erwähnten niedrigsiedenden und hochsiedenden organischen Lösungsmitteln, gibt die erhaltene Lösung zu einer Schutzkolloidlösung, wie insbesondere einer wässrigen Gelatinelösung, und dispergiert darin die Lösung mittels einer Kolloidmühle, eines Homogenisators oder durch Anwendung von Ultraschall.

Die so erhaltenen Dispersionen werden dann zur Herstellung der Schichten von farbphotographischen Aufzeichnungsmaterialien verwendet. Diese Schichten können z. B. Zwischen- oder Schutzschichten, insbesondere jedoch lichtempfindliche (blau-, grün- und rotempfindliche) Silberhalogenidemulsionsschichten sein, in denen bei der Entwicklung des belichteten Aufzeichnungsmaterials aus den entsprechenden Farbkupplern, die Blaugrün (Cyan)-, Purpur (Magenta)- und Gelbfarbstoffe gebildet werden.

Gegebenenfalls kann der Stabilisator auch in den Behandlungsbädern appliziert werden, die nach der Farbentwicklung verwendet werden, z. B. in Fixier- und/oder Waschbädern, wobei jedoch eine gewisse Löslichkeit der Verbindungen der Formel I in Alkoholen, (Methanol/Ethanol) wässrigem Alkali und/oder Wasser erforderlich ist. Wenn die Diffusionstransfermethode angewandt wird, kann der Stabilisator auch in eine Empfangsschicht eingearbeitet werden.

Die Silberhalogenidschichten können beliebige Farbkuppler, insbesondere Blaugrün-, Purpur- und Gelb-Kuppler, die zur Bildung der genannten Farbstoffe und damit der Farbbilder verwendet werden, enthalten.

Im photographischen Aufzeichnungsmaterial gemäss vorliegender Erfindung können die Stabilisatoren gemäss Formel I ausser mit den Farbkupplern zusätzlich auch mit Ultraviolettabsorbern oder anderen Lichtschutzmitteln in der gleichen Schicht kombiniert werden.

Die Silberhalogenidemulsionen enthalten als Bindemittel vorzugsweise Gelatine, gegebenenfalls im Gemisch mit anderen hochmolekularen natürlichen oder synthetischen Verbindungen.

Die Silberhalogenidemulsionen können beispielsweise Silberbromid-, Silberchlorid- oder Silberjodidemulsionen oder auch solche Emulsionen sein, die ein Gemisch von Silberhalogeniden enthalten, wie z. B. Silberbromid-jodid oder Silberchlorid-bromidemulsionen.

Die Emulsionen können chemisch sensibilisiert werden, sie können ferner übliche organische Stabilisatoren und Antischleiermittel sowie auch übliche Weichmacher, wie z. B. Glycerin, enthalten. Die Emulsionen können ferner mit den für Gelatine üblichen Härtungsmitteln gehärtet werden. Ferner können die Emulsionen übliche Giesshilfsmittel enthalten. Die Emulsionen können auf übliche Schichtträger für photographisches Aufzeichnungsmaterial aufgebracht werden.

Zur Entwicklung des farbphotographischen Aufzeichnungsmaterials können die üblichen Entwicklerbäder eingesetzt werden. Diese enthalten in der Regel eine Entwicklersubstanz des p-Phenylendiamin-Typs, einen Entwicklungsverzögerer, wie Kaliumbromid, ein Antioxydationsmittel, wie Natriumsulfit oder Hydroxylamin, und eine Base, z. B. ein Alkalihydroxid oder Alkalicarbonat. Ferner können die Entwicklungsbäder übliche Antischleiermittel und Komplexbildner enthalten.

Die erfindungsgemäss einzusetzenden Stabilisatoren eignen sich in gewissen Fällen auch zum Schutz farbphotographischer Schichten, in denen die Farbstoffe direkt in die Emulsion eingelagert werden und das Bild durch selektive Bleichung erzeugt wird.

Die Menge des Stabilisators oder der Stabilisatoren kann in weiten Grenzen schwanken und liegt etwa im Bereich von 1 bis 2 000 mg, vorzugsweise 100 bis 800 und insbesondere 200-500 mg pro $m^2$ der Schicht, in die es (sie) eingearbeitet wird (werden).

Falls das photographische Material ein UV-Strahlung absorbierendes Mittel enthält, so kann dieses mit dem Stabilisator zusammen in einer Schicht oder auch in einer benachbarten Schicht vorhanden sein. Das Gewichtsverhältnis zwischen dem Ultraviolettabsorber und dem Stabilisator der Formel I beträgt etwa (2-10) : 1, das molare Verhältnis etwa (5-20) : 1. Beispiele für Ultraviolett-Absorber sind Verbindungen vom Benzophenon-, Acrylnitril-, Thiazolidon-, Benztriazol-, Oxazol-, Thiazol- und Imidazoltyp.

Die mit dem erfindungsgemässen Aufzeichnungsmaterial durch Belichtung und Entwicklung erhaltenen Farbbilder zeigen eine sehr gute Lichtechtheit gegenüber sichtbarem und ultraviolettem Licht. Die Verbindungen der Formel I sind praktisch farblos, so dass es zu keiner Verfärbung der Bilder kommt ; ausserdem sind sie gut verträglich mit den üblichen, in den einzelnen Schichten vorhandenen photographischen Zusatzstoffen. Aufgrund ihrer guten Wirksamkeit kann man ihre Einsatzmenge herabsetzen und vermeidet so ihre Ausfällung oder ihr Auskristallisieren, wenn man sie als organische Lösung in die wässerigen Bindemittelemulsionen, die für die Herstellung photographischer Schichten verwendet werden, einarbeitet. Die einzelnen nach der Belichtung des photographischen Aufzeichnungsmaterials notwendigen Verarbeitungsschritte zur Herstellung der Farbbilder werden durch die Lichtschutzmittel nicht nachteilig beeinflusst. Ferner kann die bei blauempfindlichen Emulsionen häufig auftretende sogenannte Druckschleierbildung weitgehend zurückgedrängt werden. Diese kann z. B. auftreten, wenn auf photographische Materialien (Silberhalogenidemulsionsschichten, die sich auf einem Träger aus natürlichen oder synthetischen Materialien befinden) mechanische Beanspruchungen, z. B. Drehen, Biegen oder Reiben, während der Herstellung oder während der Behandlung vor der Entwicklung ausgeübt werden (T.H. James, The Theory of Photographic Process 4. Auflage, Macmillan, New York, N.Y. 1977, Seite 23 ff., S. 166 ff.).

Das folgende Beispeil erläutert die Erfindung näher ohne sie darauf zu beschränken.

Beispiel

0,087 g des Gelbkupplers der Formel

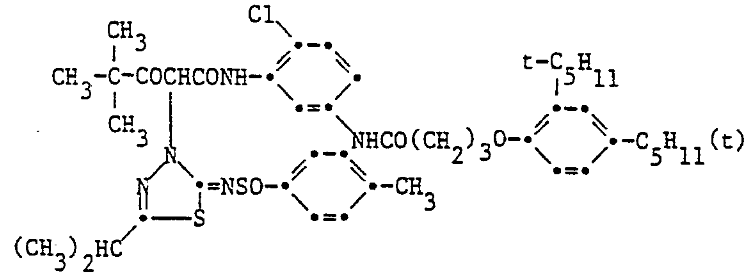

und 0,026 g eines der in den nachfolgenden Tabellen angegebenen Lichtschutzmittels werden in 2,0 ml eines Gemisches von Trikresylphosphat/Aethylacetat (1,5 g in 100 ml) gelöst. Zu dieser Lösung gibt man 7,0 ml einer 6 %igen Gelatinelösung, 0,5 ml einer 8 %igen Lösung des Netzmittels der Formel

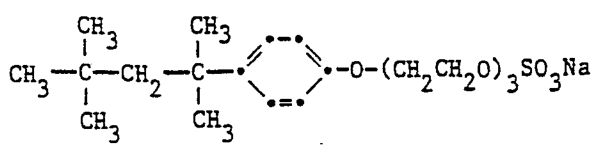

in Isopropanol/Wasser (3 : 4) und 0,5 ml Wasser und emulgiert mit Ultraschall bei einer Leistung von 100 Watt während 5 Minuten.

Zu 2,5 ml der so erhaltenen Emulsion gibt man 2,0 ml einer Silberbromid-Emulsion mit einem Silbergehalt von 6,0 g pro Liter, 0,7 ml einer 1 %igen wässerigen Lösung des Härters der Formel

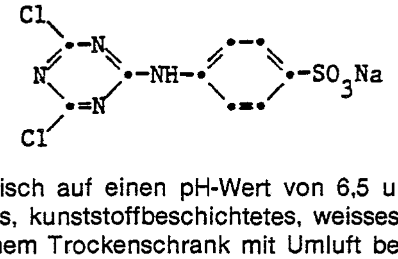

und 3,8 ml Wasser, stellt das Gemisch auf einen pH-Wert von 6,5 und vergiesst es auf ein auf eine Glasplatte aufgezogenes substriertes, kunststoffbeschichtetes, weisses Papier.

Nach dem Erstarren wird in einem Trockenschrank mit Umluft bei Raumtemperatur getrocknet.

Nach 7 Tagen werden auf 35 × 180 mm geschnittene Proben hinter einem Stufenkeil mit 3 000 Lux · s belichtet und anschliessend im Ektaprint 2®-Prozess der Firma Kodak verarbeitet.

Die so erhaltenen Gelbkeile werden in einem Atlas Weather-Ometer mit einer 2 500 W-Xenonlampe mit total 42 $kJoule/cm^2$ bestrahlt (eine Vergleichsprobe enthält kein Lichtschutzmittel).

In der Tabelle sind die prozentualen Farbdichteabnahmen bei einer ursprünglichen Dichte von 1,0 enthalten.

| Lichtschutzmittel Verbindung Nr. | Farbdichteverlust in Prozent (Remission) |
|---|---|
|  | 49 |
| 1 | 26 |
| 2 | 32 |
| 5 | 30 |
| 7 | 32 |
| 10 | 30 |
| 11 | 30 |

**Patentansprüche**

1. Farbphotographisches Aufzeichnungsmaterial, das in mindestens einer lichtempfindlichen Silberhalogenid-Emulsionsschicht, einer Zwischenschicht, einer Bildempfangsschicht und/oder einer Schutzschicht als Stabilisator mindestens eine Polyalkylpiperidinverbindung der Formel I enthält,

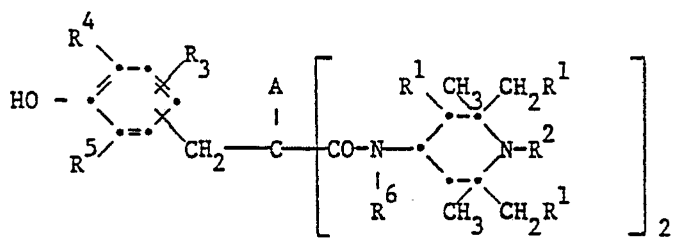

(I)

worin

$R^1$ Wasserstoff oder Methyl ist,

$R^2$ Hydroxyl, $C_1$-$C_{12}$-Alkyl, $C_3$-$C_6$-Alkenyl, $C_3$-$C_4$-Alkinyl, $C_7$-$C_{12}$-Aralkyl, Glycidyl, durch Halogen, Cyan, $—COOR^7$ oder $—CON(R^8)(R^9)$ substituiertes $C_1$-$C_4$-Alkyl, eine Gruppe $—CO—R^{10}$, $—CO—OR^7$, $—CO—N(R^8)(R^9)$, $—CH_2$-$CH(R^{11})—OR^{12}$, $—SO—R^{13}$, $—SO_2—R^{13}$, $—OR^7$ oder $—OOC—R^{10}$ bedeutet,

$R^3$ Wasserstoff oder Methyl ist,

$R^4$ $C_1$-$C_8$-Alkyl, $C_5$-$C_8$-Cycloalkyl, $C_7$-$C_9$-Phenylalkyl, Phenyl oder $C_7$-$C_{10}$-Alkylphenyl und

$R^5$ Wasserstoff, $C_1$-$C_8$-Alkyl, $C_5$-$C_8$-Cycloalkyl, $C_7$-$C_9$-Phenylalkyl, Phenyl oder $C_7$-$C_{10}$-Alkylphenyl bedeutet,

$R^6$ Wasserstoff, $C_1$-$C_{18}$-Alkyl, $C_3$-$C_6$-Alkenyl, $C_3$-$C_{12}$-Cycloalkyl, $C_7$-$C_{12}$-Aralkyl, $C_7$-$C_{10}$-Alkylphenyl, durch $—OR^8$, $—N(R^9)_2$ oder eine Gruppe

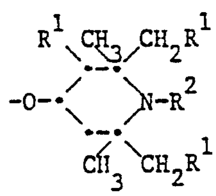

substituiertes $C_2$-$C_4$-Alkyl bedeutet,

$R^7$ $C_1$-$C_{12}$-Alkyl, Allyl, Cyclohexyl oder Benzyl bedeutet,

$R^8$ $C_1$-$C_{12}$-Alkyl, Allyl, Cyclohexyl, Benzyl oder Phenyl bedeutet,

$R^9$ Wasserstoff, $C_1$-$C_{12}$-Alkyl, Allyl, Cyclohexyl oder Benzyl bedeutet oder

$R^8$ und $R^9$ zusammen mit dem N-Atom, an das sie gebunden sind, einen 5- oder 6-gliedrigen heterocyclischen Ring bilden,

$R^{10}$ Wasserstoff, $C_1$-$C_{12}$-Alkyl, $C_2$-$C_6$-Alkenyl, Chlormethyl, $C_5$-$C_8$-Cycloalkyl, $C_7$-$C_{12}$-Aralkyl, Phenyl, $C_7$-$C_{10}$-Alkylphenyl oder durch 1 oder 2 $C_1$-$C_4$-Alkylgruppen und eine Hydroxygruppe substituiertes Phenyl, Phenylmethyl oder Phenylethyl bedeutet,

$R^{11}$ Wasserstoff, $C_1$-$C_4$-Alkyl, $C_2$-$C_{13}$-Alkoxymethyl, Phenyl oder Phenoxymethyl bedeutet,
$R^{12}$ Wasserstoff, $C_1$-$C_{12}$-Alkyl, —CO—$R^{10}$ oder —CO—N($R^8$) ($R^9$) bedeutet,
$R^{13}$ $C_1$-$C_{12}$-Alkyl, Phenyl oder $C_7$-$C_{18}$-Alkylaryl bedeutet,
A Wasserstoff, $C_1$-$C_{18}$-Alkyl, durch mindestens eine der Gruppen —$OR^{14}$, —$SR^{15}$, —CN, —CO—X—$R^{16}$, —O—CO—$R^{17}$ oder —P(O) $(OR^{18})_2$ substituiertes $C_1$-$C_{10}$-Alkyl, durch —O—, —S—, —SO— oder —$SO_2$— unterbrochenes $C_2$-$C_{20}$-Alkyl, $C_3$-$C_{18}$-Alkenyl, $C_3$-$C_8$-Alkinyl, $C_3$-$C_{12}$-Cycloalkyl, $C_6$-$C_{10}$-Alkylcycloalkyl, $C_6$-$C_{10}$-Cycloalkylalkyl, $C_7$-$C_{12}$-Aralkyl, $C_8$-$C_{16}$-Alkylaralkyl, Phenyl, eine Gruppe —CN, —CO—$R^{20}$, —$SO_2$—$R^{21}$, —P(O) ($OR^{18}$) oder eine der folgenden Gruppen bedeutet

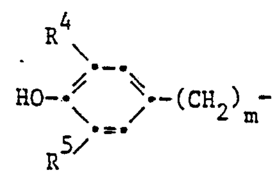 , 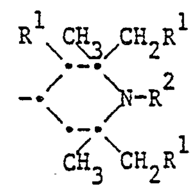

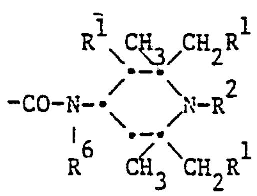 , 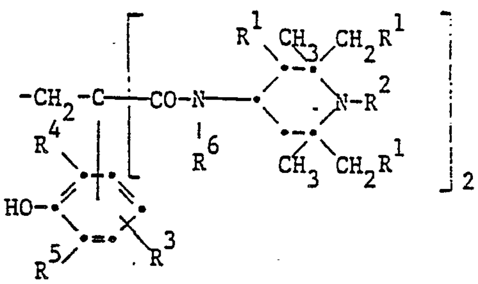

worin
X —O— oder —N($R^{19}$)— bedeutet,
$R^{14}$ Phenyl, Benzyl oder Cyclohexyl,
$R^{15}$ Phenyl oder $C_7$-$C_9$-Phenylalkyl,
$R^{16}$ $C_1$-$C_{18}$-Alkyl, $C_5$-$C_8$-Cycloalkyl und
$R^{17}$ $C_1$-$C_{18}$-Alkyl, $C_5$-$C_8$-Cycloalkyl, Phenyl, $C_7$-$C_9$-Phenylalkyl oder eine Gruppe

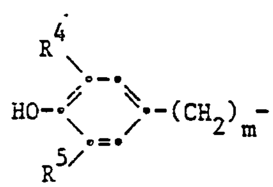

bedeutet,
$R^{18}$ $C_1$-$C_8$ Alkyl, Allyl oder Phenyl ist
$R^{19}$ Wasserstoff, $C_1$-$C_{18}$-Alkyl, Allyl, Cyclohexyl, Benzyl oder Phenyl bedeutet oder zusammen mit $R^{16}$ und dem N-Atom einen 5- oder 6-gliedrigen heterocyclischen Ring bildet,
$R^{20}$ Wasserstoff, $C_1$-$C_{18}$-Alkyl, $C_2$-$C_6$-Alkenyl, $C_5$-$C_8$-Cycloalkyl, Phenyl, $C_7$-$C_{10}$-Alkylphenyl, $C_7$-$C_9$-Phenylalkyl und
$R^{21}$ $C_1$-$C_{18}$-Alkyl, Cyclohexyl, Phenyl, Naphthyl, $C_7$-$C_{18}$-Alkylaryl, $C_7$-$C_9$-Phenylalkyl oder eine Gruppe der Formel

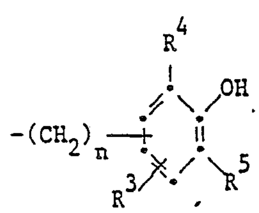

bedeutet,
m null, 1 oder 2 ist und
n null, 1, 2 oder 3 ist.

2. Farbphotographisches Aufzeichnungsmaterial gemäss Anspruch 1, dadurch gekennzeichnet, dass der Stabilisator eine Verbindung der Formel II ist

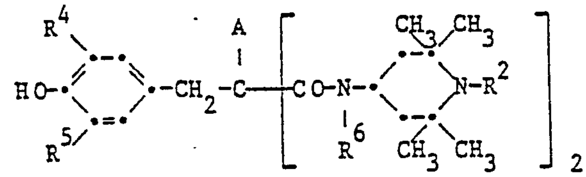

(II)

worin

$R^2$ Hydroxy, $C_1$-$C_4$-Alkyl, $C_3$-$C_4$-Alkenyl, Propargyl, Benzyl, Glycidyl, durch Halogen, Cyan, —$COOR^7$, —$CON(R^8)$ $(R^9)$ substituiertes $C_1$-$C_4$-Alkyl, eine Gruppe —CO—$R^{10}$, —CO—$OR^7$, —CO—$N(R^8)$ $(R^9)$, —$CH_2$—$CH(R^{11})$—$OR^{12}$, —SO—$R^{13}$, —$SO_2$—$R^{13}$, —$OR^7$ oder —OOC—$R^{10}$ bedeutet

$R^4$ $C_1$-$C_4$-Alkyl und $R_5$ Wasserstoff oder $C_1$-$C_4$-Alkyl ist,

$R^6$ Wasserstoff, $C_1$-$C_{12}$-Alkyl, $C_3$-$C_7$-Alkoxyalkyl, Cyclohexyl oder eine Gruppe der Formel

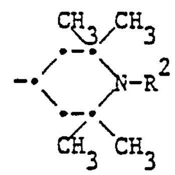

bedeutet,

$R^7$ $C_1$-$C_8$-Alkyl, Benzyl oder Cyclohexyl bedeutet,

$R^8$ $C_1$-$C_8$-Alkyl, Cyclohexyl oder Phenyl bedeutet,

$R^9$ Wasserstoff oder $C_1$-$C_8$-Alkyl bedeutet oder $R^8$ und $R^9$ zusammen mit dem N-Atom einen Piperidin- oder Morpholinring bilden,

$R^{10}$ $C_1$-$C_4$-Alkyl, $C_2$-$C_3$-Alkenyl, Chlormethyl, Phenyl, Benzyl oder eine Gruppe der Formel

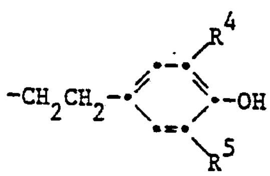

bedeutet,

$R^{11}$ Wasserstoff, Methyl oder Phenyl ist,

$R^{12}$ Wasserstoff, $C_1$-$C_8$-Alkyl, —CO—$R^{10}$ oder —CO—$N(R^8)$ $(R^9)$ bedeutet,

$R^{13}$ $C_1$-$C_4$-Alkyl, Phenyl oder p-Tolyl bedeutet und

A Wasserstoff, $C_1$-$C_8$-Alkyl, Cyan, Allyl oder Benzyl bedeutet.

3. Farbphotographisches Aufzeichnungsmaterial gemäss Anspruch 2, dadurch gekennzeichnet, dass der Stabilisator eine Verbindung der Formel II ist, worin

$R^2$ Hydroxy, Methyl, Allyl, Benzyl, 2-Hydroxyethyl, Acetyl, Acryloyl, Methoxy, Acetoxy oder eine Gruppe —CO—$N(R^8)$ $(R^9)$

bedeutet,

$R^4$ tert.-Butyl und $R^5$ Methyl oder tert.-Butyl ist,

$R^6$ Wasserstoff oder $C_1$-$C_8$-Alkyl ist,

$R^8$ $C_1$-$C_8$-Alkyl, Phenyl oder Cyclohexyl und $R^9$ Wasserstoff oder $C_1$-$C_8$-Alkyl ist und

A Wasserstoff, $C_1$-$C_8$-Alkyl oder Benzyl bedeutet.

4. Farbphotographisches Aufzeichnungsmaterial gemäss Anspruch 1, dadurch gekennzeichnet, dass es neben einem Stabilisator der Formel I ein Lichtschutzmittel aus der Klasse der Ultraviolett-Absorber enthält.

5. Farbphotographisches Aufzeichnungsmaterial gemäss Anspruch 1, gekennzeichnet durch einen Gehalt an 1 bis 2 000, vorzugsweise 100 bis 800 mg pro $m^2$ an Verbindung der Formel I.

6. Verwendung der in Anspruch 1 definierten Verbindungen der Formel I als Stabilisatoren für farbphotographische Aufzeichnungsmaterialien.

**Claims**

1. A recording material for colour photography which in at least one light-sensitive silver halide emulsion layer, an intermediate layer, an image-receiving layer and/or a protective layer contains, as a stabiliser, at least one polyalkylpiperidine compound of the formula I

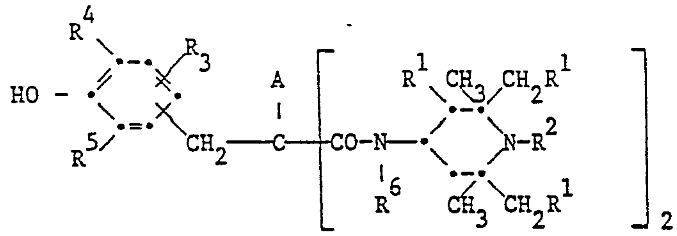

(I)

in which

$R^1$ is hydrogen or methyl,

$R^2$ is hydroxy, $C_1$-$C_{12}$-alkyl, $C_3$-$C_6$-alkenyl, $C_3$-$C_4$-alkynyl, $C_7$-$C_{12}$-aralkyl, gycidyl, $C_1$-$C_4$-alkyl which is substituted by halogen, cyano, —COOR$^7$ or —CON($R^8$)($R^9$), or is a —CO—$R^{10}$, —CO—OR$^7$, —CO—N—($R^8$)($R^9$), —$CH_2$—CH($R^{11}$)—OR$^{12}$, —SO—$R^{13}$, —$SO_2$—$R^{13}$, —OR$^7$ or —OOC—$R^{10}$ group,

$R^3$ is hydrogen or methyl,

$R^4$ is $C_1$-$C_8$-alkyl, $C_5$-$C_8$-cycloalkyl, $C_7$-$C_9$-phenylalkyl, phenyl or $C_7$-$C_{10}$-alkylphenyl,

$R^5$ is hydrogen, $C_1$-$C_8$-alkyl, $C_5$-$C_8$-cycloalkyl, $C_7$-$C_9$-phenylalkyl, phenyl or $C_7$-$C_{10}$-alkylphenyl,

$R^6$ is hydrogen, $C_1$-$C_{18}$-alkyl, $C_3$-$C_6$-alkenyl, $C_3$-$C_{12}$-cycloalkyl, $C_7$-$C_{12}$-aralkyl, $C_7$-$C_{10}$-alkylphenyl, or $C_2$-$C_4$-alkyl which is substituted by —OR$^8$, —N($R^9$)$_2$ or a group of the formula

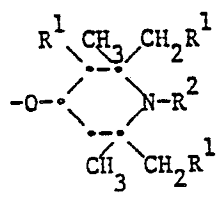

$R^7$ is $C_1$-$C_{12}$-alkyl, allyl, cyclohexyl or benzyl,

$R^8$ is $C_1$-$C_{12}$-alkyl, allyl, cyclohexyl, benzyl or phenyl,

$R^9$ is hydrogen, $C_1$-$C_{12}$-alkyl, allyl, cyclohexyl or benzyl, or

$R^8$ and $R^9$, together with the N atom to which they are bonded, form a 5- or 6-membered heterocyclic ring,

$R^{10}$ is hydrogen, $C_1$-$C_{12}$-alkyl, $C_2$-$C_6$-alkenyl, chloromethyl, $C_5$-$C_8$-cycloalkyl, $C_7$-$C_{12}$-aralkyl, phenyl, $C_7$-$C_{10}$-alkylphenyl, or is phenyl, phenylmethyl or phenylethyl, each of which is subtituted by 1 or 2 $C_1$-$C_4$ alkyl groups and a hydroxyl group,

$R^{11}$ is hydrogen, $C_1$-$C_4$-alkyl, $C_2$-$C_{13}$-alkoxymethyl, phenyl or phenoxymethyl,

$R^{12}$ is hydrogen, $C_1$-$C_{12}$-alkyl, —CO—$R^{10}$ or —CO—N($R^8$)($R^9$),

$R^{13}$ is $C_1$-$C_{12}$-alkyl, phenyl or $C_7$-$C_{18}$-alkylaryl,

A is hydrogen, $C_1$-$C_{18}$-alkyl, $C_1$-$C_{10}$-alkyl which is substituted by at least one of the groups —OR$^{14}$, —SR$^{15}$, —CN, —CO—X—$R^{16}$, —O—CO—$R^{17}$ or —P(O)(OR$^{18}$)$_2$, or is $C_2$—$C_{20}$—alkyl which is interrupted by —O—, —S—, —SO— or —$SO_2$—, or is $C_3$-$C_{18}$-alkenyl, $C_3$-$C_8$-alkynyl, $C_3$-$C_{12}$-cycloalkyl, $C_6$-$C_{10}$-alkylcycloalkyl, $C_6$-$C_{10}$-cycloalkylalkyl, $C_7$-$C_{12}$-aralkyl, $C_8$-$C_{16}$-alkylaralkyl, phenyl, a —CN, —CO—$R^{20}$, —$SO_2$—$R^{21}$ or —P(O)(OR$^{18}$) group or one of the following groups

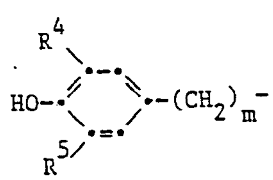

,

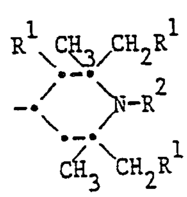

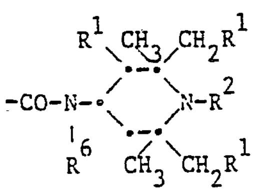

,

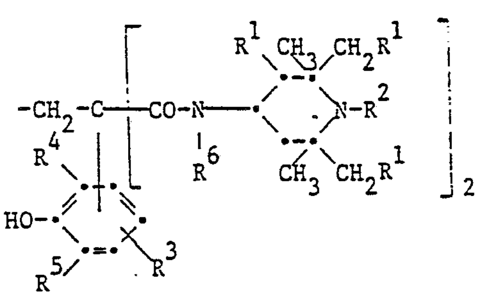

in which formulae

X is —O— or —N($R^{19}$)—,

$R^{14}$ is phenyl, benzyl or cyclohexyl,

$R^{15}$ is phenyl or $C_7$-$C_9$-phenylalkyl,

$R^{16}$ is $C_1$-$C_{18}$-alkyl, $C_5$-$C_8$-cycloalkyl and

$R^{17}$ is $C_1$-$C_{18}$-alkyl, $C_5$-$C_8$-cycloalkyl, phenyl, $C_7$-$C_9$-phenylalkyl or a group of the formula

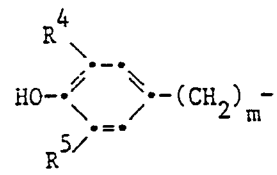

$R^{18}$ is $C_1$-$C_8$-alkyl, allyl or phenyl,

$R^{19}$ is hydrogen, $C_1$-$C_{18}$-alkyl, allyl, cyclohexyl, benzyl or phenyl or, together with $R^{16}$ and the N atom, forms a 5- or 6-membered heterocyclic ring,

$R^{20}$ is hydrogen, $C_1$-$C_{18}$-alkyl, $C_2$-$C_6$-alkenyl, $C_5$-$C_8$-cycloalkyl, phenyl, $C_7$-$C_{10}$-alkylphenyl or $C_7$-$C_9$-phenylalkyl,

$R^{21}$ is $C_1$-$C_{18}$-alkyl, cyclohexyl, phenyl, naphthyl, $C_7$-$C_{18}$-alkylaryl, $C_7$-$C_9$-phenylalkyl or a group of the formula

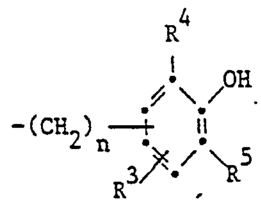

m is zero, 1 or 2, and

n is zero, 1, 2 or 3.

2. A recording material for colour photography according to claim 1, wherein the stabiliser is a compound of the formula II

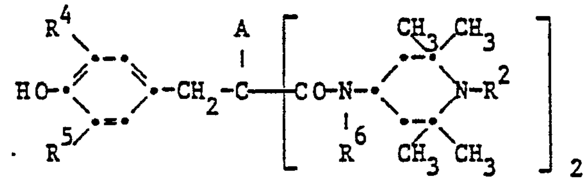

(II)

in which

$R^2$ is hydroxy, $C_1$-$C_4$-alkyl, $C_3$-$C_4$-alkenyl, propargyl, benzyl, glycidyl, $C_1$-$C_4$-alkyl which is substituted by halogen, cyano, —COOR$^7$ or —CON($R^8$)($R^9$), or is a —CO—$R^{10}$, —CO—OR$^7$, —CO—N($R^8$)($R^9$), —$CH_2$—CH($R^{11}$)—OR$^{12}$, —SO—$R^{13}$, —$SO_2$—$R^{13}$, —OR$^7$ or —OOC—$R^{10}$ group,

$R^4$ is $C_1$-$C_4$-alkyl,

$R^5$ is hydrogen or $C_1$-$C_4$-alkyl,

$R^6$ is hydrogen, $C_1$-$C_{12}$-alkyl, $C_3$-$C_7$-alkoxyalkyl, cyclohexyl or a group of the formula

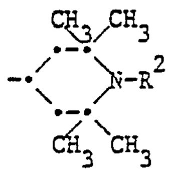

$R^7$ is $C_1$-$C_8$-alkyl, benzyl or cyclohexyl,

$R^8$ is $C_1$-$C_8$-alkyl, cyclohexyl or phenyl,

$R^9$ is hydrogen or $C_1$-$C_8$-alkyl, or $R^8$ and $R^9$, together with the N atom, form a piperidine or morpholine ring,

$R^{10}$ is $C_1$-$C_4$-alkyl, $C_2$-$C_3$-alkenyl, chloromethyl, phenyl, benzyl or a group of the formula

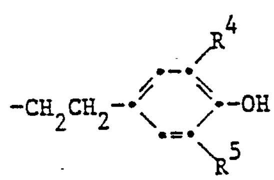

$R^{11}$ is hydrogen, methyl or phenyl,

$R^{12}$ is hydrogen, $C_1$-$C_8$-alkyl, —CO—$R^{10}$ or —CO—N($R^8$)($R^9$),

$R^{13}$ is $C_1$-$C_4$-alkyl, phenyl or p-tolyl, and

A is hydrogen, $C_1$-$C_8$-alkyl, cyano, allyl or benzyl.

3. A recording material for colour photography according to claim 2, wherein the stabiliser is a compound of the formula II in which

$R^2$ is hydroxy, methyl, allyl, benzyl, 2-hydroxyethyl, acetyl, acryloyl, methoxy, acetoxy or a —CO—N($R^8$)($R^9$) group,

$R^4$ is tert-butyl,

$R^5$ is methyl or tert-butyl,

$R^6$ is hydrogen or $C_1$-$C_8$-alkyl,

$R^8$ is $C_1$-$C_8$-alkyl, phenyl or cyclohexyl,

$R^9$ is hydrogen or $C_1$-$C_8$-alkyl, and

A is hydrogen, $C_1$-$C_8$-alkyl or benzyl.

4. A recording material for colour photography according to claim 1, which contains, in addition to a stabiliser of the formula I, a light stabiliser of the ultraviolet absorber class.

5. A recording material for colour photography according to claim 1, which contains 1 or 2,000, preferably 100 to 800, mg of compound of the formula I per $m^2$.

6. Use of a compound of the formula I defined in claim 1 as a stabiliser for recording materials for colour photography.

**Revendications**

1. Matière d'enregistrement pour photographie en couleurs, qui contient, dans au moins une couche d'émulsion d'halogénure d'argent photosensible, une couche intermédiaire, une couche de réception de l'image et/ou une couche protectrice, comme stabilisant, au moins un composé polyalkyl-pipéridinique répondant à la formule I :

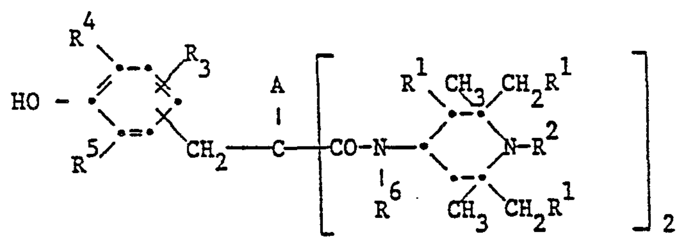

(I)

dans laquelle

$R^1$ représente l'hydrogène ou un méthyle,

$R^2$ représente un hydroxy, un alkyle en $C_1$-$C_{12}$, un alcényle en $C_3$-$C_6$, un alcynyle en $C_3$ ou $C_4$, un aralkyle en $C_7$-$C_{12}$, un glycidyle, un alkyle en $C_1$-$C_4$ porteur d'un halogène, d'un cyano, d'un radical $COOR^7$ ou d'un radical —CON($R^8$)($R^9$), ou un radical —CO—$R^{10}$, —CO—$OR^7$, —CO—N($R^8$)($R^9$), —$CH_2$—CH($R^{11}$)—$OR^{12}$, —SO—$R^{13}$, —$SO_2$—$R^{13}$, —$OR^7$ ou —OOC—$R^{10}$,

$R^3$ représente l'hydrogène ou un méthyle,

$R^4$ représente un alkyle en $C_1$-$C_8$, un cycloalkyle en $C_5$-$C_8$, un phénylalkyle en $C_7$-$C_9$, un phényle ou un alkylphényle en $C_7$-$C_{10}$,

$R^5$ représente l'hydrogène, un alkyle en $C_1$-$C_8$, un cycloalkyle en $C_5$-$C_8$, un phénylalkyle en $C_7$-$C_9$, un phényle ou un alkylphényle en $C_7$-$C_{10}$,

$R^6$ représente l'hydrogène, un alkyle en $C_1$-$C_{18}$, un alcényle en $C_3$-$C_6$, un cycloalkyle en $C_3$-$C_{12}$, un aralkyle en $C_7$-$C_{12}$, un alkylphényle en $C_7$-$C_{10}$ ou un alkyle en $C_2$-$C_4$ qui porte un radical —$OR^8$, un radical —N($R^9$)$_2$ ou un radical :

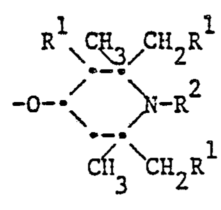

$R^7$ représente un alkyle en $C_1$-$C_{12}$, un allyle, un cyclohexyle ou un benzyle,

$R^8$ représente un alkyle en $C_1$-$C_{12}$, un allyle, un cyclohexyle un benzyle ou un phényle,

$R^9$ représente l'hydrogène, un alkyle en $C_1$-$C_{12}$, un allyle, un cyclohexyle ou un benzyle,

ou encore

$R^8$ et $R^9$ forment ensemble et avec l'atome d'azote auquel ils sont liés un noyau hétérocyclique à 5 ou 6 maillons,

$R^{10}$ représente l'hydrogène, un alkyle en $C_1$-$C_{12}$, un alcényle en $C_2$-$C_6$, un chlorométhyle, un cycloalkyle en $C_5$-$C_8$, un aralkyle en $C_7$-$C_{12}$, un phényle, un alkylphényle en $C_7$-$C_{10}$ ou un radical phényle, phénylméthyle ou phényléthyle, porteur d'un ou deux radicaux alkyles en $C_1$-$C_4$ ou d'un radical hydroxy,

$R^{11}$ représente l'hydrogène, un alkyle en $C_1$-$C_4$, un alcoxyméthyle en $C_2$-$C_{13}$, un phényle ou un phénoxyméthyle,

$R^{12}$ représente l'hydrogène, un alkyle en $C_1$-$C_{12}$ ou un radical —CO—$R^{10}$ ou —CO—N($R^8$)($R^9$),

$R^{13}$ représente un alkyle en $C_1$-$C_{12}$, un phényle ou un alkylaryle en $C_7$-$C_{18}$, et

A représente l'hydrogène, un alkyle en $C_1$—$C_{18}$, un alkyle en $C_1$-$C_{10}$ porteur d'au moins un des radicaux $OR^{14}$, —$SR^{15}$, —CN, —CO—X—$R^{16}$, —O—CO—$R^{17}$ et —P(O)($OR^{18}$)$_2$, un alkyle en $C_2$-$C_{20}$ interrompu par —O—, —S—, —SO— ou —$SO_2$—, un alcényle en $C_3$-$C_{18}$, un alcynyle en $C_3$-$C_8$, un cycloalkyle en $C_3$-$C_{12}$, un alkylcycloalkyle en $C_6$-$C_{10}$, un cycloalkyl-alkyle en $C_6$-$C_{10}$, un aralkyle en $C_7$-$C_{12}$, un alkyl-aralkyle en $C_8$-$C_{16}$, un phényle, un radical —CN, —CO—$R^{20}$, —$SO_2$—$R^{21}$ ou —P(O)($OR^{18}$), ou un des radicaux suivants :

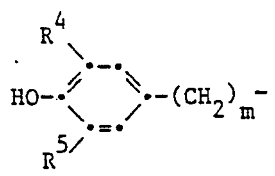
,
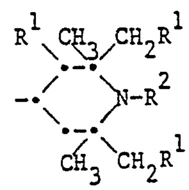

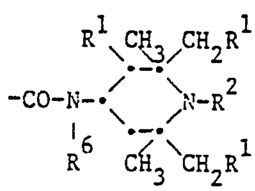
,
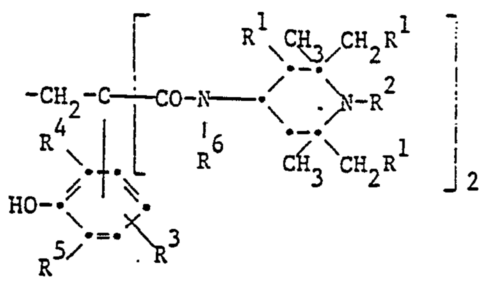

radicaux dans lesquels :

X représente —O— ou —N($R^{19}$)—,

$R^{14}$ représente un phényle, un benzyle ou un cyclohexyle,

$R^{15}$ représente un phényle ou un phénylalkyle en $C_7$-$C_9$,

$R^{16}$ représente un alkyle en $C_1$-$C_{18}$ ou un cycloalkyle en $C_5$-$C_8$,

$R^{17}$ représente un alkyle en $C_1$-$C_{18}$, un cycloalkyle en $C_5$-$C_8$, un phényle, un phénylalkyle en $C_7$-$C_9$ ou un radical :

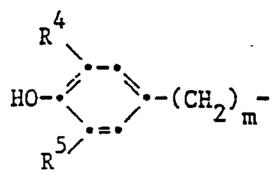

$R^{18}$ représente un alkyle en $C_1$-$C_8$, un allyle ou un phényle,

$R^{19}$ représente l'hydrogène, un alkyle en $C_1$-$C_{18}$, un allyle, un cyclohexyle, un benzyle ou un phényle, ou forme avec $R^{16}$ et l'atome d'azote un noyau hétérocyclique à 5 ou 6 maillons,

$R^{20}$ représente l'hydrogène, un alkyle en $C_1$-$C_{18}$, un alcényle en $C_2$-$C_6$, un cycloalkyle en $C_5$-$C_8$, un phényle, un alkylphényle en $C_7$-$C_{10}$ ou un phénylalkyle en $C_7$-$C_9$,

$R^{21}$ représente un alkyle en $C_1$-$C_{18}$, un cyclohexyle, un phényle, un naphtyle, un alkylaryle en $C_7$-$C_{18}$, un phénylalkyle en $C_7$-$C_9$ ou un radical de formule :

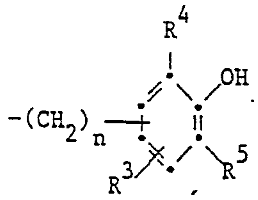

m est égal à zéro, à 1 ou à 2 et

n est égal à zéro, à 1, à 2 ou à 3.

2. Matière d'enregistrement pour photographie en couleurs selon la revendication 1, matière caractérisée en ce que le stabilisant est un composé répondant à la formule II :

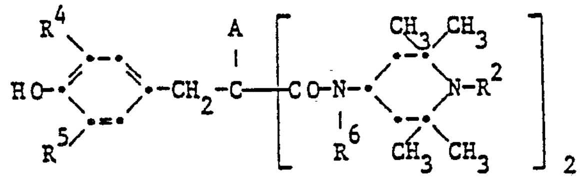

(II)

dans laquelle

$R^2$ représente un hydroxy, un alkyle en $C_1$-$C_4$, un alcényle en $C_3$ ou $C_4$, un propargyle, un benzyle, un glycidyle, un alkyle en $C_1$-$C_4$ porteur d'un halogène, d'un cyano ou d'un radical $-COOR^7$ ou $-CON(R^8)(R^9)$, ou un radical $-CO-R^{10}$, $-CO-OR^7$, $-CO-N(R^8)(R^9)$, $-CH_2-CH(R^{11})-OR^{12}$, $-SO-R^{13}$, $-SO_2-R^{13}$, $-OR^7$ ou $-OOC-R^{10}$,

$R^4$ représente un alkyle en $C_1$-$C_4$,

$R^5$ représente l'hydrogène ou un alkyle en $C_1$-$C_4$,

$R^6$ représente l'hydrogène, un alkyle en $C_1$-$C_{12}$, un alcoxyalkyle en $C_3$-$C_7$, un cyclohexyle ou un radical de formule :

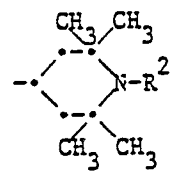

$R^7$ représente un alkyle en $C_1$-$C_8$, un benzyle ou un cyclohexyle,

$R^8$ représente un alkyle en $C_1$-$C_8$, un cyclohexyle ou un phényle,

$R^9$ représente l'hydrogène ou un alkyle en $C_1$-$C_8$, ou encore $R^8$ et $R^9$ forment ensemble et avec l'atome d'azote un noyau de pipéridine ou de morpholine,

$R^{10}$ représente un alkyle en $C_1$-$C_4$, un alcényle en $C_2$ ou $C_3$, un chlorométhyle, un phényle, un benzyle ou un radical de formule :

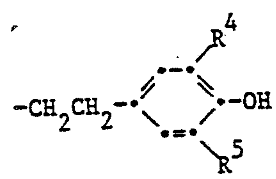

$R^{11}$ représente l'hydrogène, un méthyle ou un phényle,

$R^{12}$ représente l'hydrogène, un alkyle en $C_1$-$C_8$ ou un radical $-CO-R^{10}$ ou $-CO-N(R^8)(R^9)$,

$R^{13}$ représente un alkyle en $C_1$-$C_4$, un phényle ou un p-tolyle et

A représente l'hydrogène, un alkyle en $C_1$-$C_8$, un cyano, un allyle ou un benzyle.

3. Matière d'enregistrement pour photographie en couleurs selon la revendication 2, matière caractérisée en ce que le stabilisant est un composé de formule II dans lequel :

$R^2$ représente un hydroxy, un méthyle, un allyle, un benzyle, un hydroxy-2 éthyle, un acétyle, un acryloyle, un méthoxy, un acétoxy ou un radical $-CO-N(R^8)(R^9)$,

$R^4$ représente un tert-butyle,

$R^5$ représente un méthyle ou un tert-butyle,

$R^6$ représente l'hydrogène ou un alkyle en $C_1$-$C_8$,

$R^8$ représente un alkyle en $C_1$-$C_8$, un phényle ou un cyclohexyle,

$R^9$ représente l'hydrogène ou un alkyle en $C_1$-$C_8$ et

A représente l'hydrogène, un alkyle en $C_1$-$C_8$ ou un benzyle.

4. Matière d'enregistrement pour photographie en couleurs selon la revendication 1, matière caractérisée en ce qu'elle contient, en plus d'un stabilisant de formule I, un stabilisant à la lumière appartenant à la catégorie des absorbeurs de rayons ultraviolets.

5. Matière d'enregistrement pour photographie en couleurs selon la revendication 1, matière caractérisée en ce qu'elle contient, par $m^2$, de 1 à 2 000 mg d'un composé de formule I, de préférence de 100 à 800 mg.

6. Application des composés de formule I définis à la revendication 1 comme stabilisants pour des matières d'enregistrement pour photographie en couleurs.